Europäisches Patentamt

European Patent Office

Office européen des brevets

(11) Publication number: **0 346 294**
**A1**

# (12) EUROPEAN PATENT APPLICATION

(21) Application number: **89830229.4**

(22) Date of filing: **23.05.89**

(51) Int. Cl.⁴: **A 61 F 2/32**

(30) Priority: **23.05.88 IT 347188**

(43) Date of publication of application:
**13.12.89 Bulletin 89/50**

(84) Designated Contracting States:
**DE ES FR GB GR SE**

(71) Applicant: **Impallomeni, Carlo, Dott.**
**via P. To Salvo, 10**
**Messina (IT)**

(72) Inventor: **Impallomeni, Carlo, Dott.**
**via P. To Salvo, 10**
**Messina (IT)**

(74) Representative: **Dall'Olio, Giancarlo**
**INVENTION s.n.c. Via Arienti 26**
**I-40124 Bologna (IT)**

(54) **Method and device for the retention of debris set free by a total hip endoprosthesis.**

㊗ The method involves the positioning and fixing of a cup 2,12, forming an artificial acetabular cavity 5,15 within the pelvic cotyloid cavity of the subject with a prosthesis, the introduction and fixing in the femoral canal of a shaft 7, to which an artificial femoral head 6 is fastened, after resection of the natural head and neck of the femur, the fitting of a cup 2,12 in a sheath onto the neck 11 of the shaft 7. As an alternative, the sheath may already be fastened to the cup 12 or to an insert 14 containing the acetabular cavity 25 and designed to be fitted inside the metal cup 2.

Fig. 2

**Description**

## METHOD AND DEVICE FOR THE RETENTION OF DEBRIS LOST BY JOINTS OF TOTAL HIP PROSTHESES

This invention regards an orthopedic prosthesis. The socalled total hip prostheses are well known and have been used for some time; they generally consist of a number of components which vary according to the functional characteristics required.

A first component, known to the technicians of the sector as the "cup", may be produced in a single piece of polyethylene or in metal, using different types of alloys and include, on its inner surface, a fixed or mobile polyethylene insert.

Depending on its design, the cup may be fixed by screw cementing or "press-fitting" (i.e. fitted with extreme precision in a matching seat accurately prepared in the pelvis, so that it is firmly attached) to the corresponding bone part.

The metal cups may contain an internal part, in polyethylene, fixed to them and appropriately shaped, forming an artificial acetabular cavity, or they may have a cavity designed to contain a polyethylene insert shaped so that it adapts and fastens itself to the metal cup, on the one side, and forms the aforementioned cavity on the other.

The insert is correctly positioned after the metal cup is fixed.

The all-polyethylene cups already contain the acetabular cavity and are cemented into the prepared bone cavity. Finally, a femoral head and shaft are envisaged.

In some cases the femoral head is an integral part of the shaft and they are both produced in metal alloy.

In other cases, the femoral head is produced in alloy or in ceramic and is fixed to the shaft after the latter has been positioned in the femoral canal and the remaining parts of the prosthesis have been placed in their respective seats.

One of the main problems still arising today, for orthopedic surgeons and bioengineers, with total hip prostheses is that represented by the formation of debris around the contact surfaces between the spherical head and hemispherical cavity of the polyethylene cup or the insert which houses it, as a consequence of the inevitable friction occurring between them.

These fragments, released as mentioned above, spread throughout the surrounding area and, since they are not biologically inert, may stimulate a series of processes by which the organism attempts to eliminate them. The fragments may, on the one hand, be carried away (with various mechanisms) and, on the other, in their turn locally initiate a reactive biological process affecting, amongst others, even the bone tissue, frequently leading to phenomena by which they are reabsorbed with the consequent mobilization of the prosthesis.

As a result of the latter problem it is frequently necessary to remove and/or replace the prosthesis, with all the relative problems involved: e.g. those arising from another invasive operation.

The aim of the present invention is that of proposing a method which, by applying a device to the known types of prostheses, isolates the area where these debris are formed from the rest of the organism, so that the debris, whose formation is at present inevitable, cannot affect the local biological environment, or be transported to other parts of the body even far away from the hip.

Another aim of this invention is that of offering a device which, while it retains the debris, does not impede the lubrication of the articular prosthetic components, normally carried out by biological liquids.

Finally, an aim of the invention is to offer the abovementioned device, produced in a technically reliable and simple to produce form, without causing disturbance or other damaging effects to the subject with the prosthesis.

The above aims are achieved by a method for retaining debris which is detached from total hip prosthetic joints, the latter being of the type consisting of: a cup, designed to be fixed inside the cotyloid cavity of the pelvis of the subject fitted with the said prosthesis, with an artificial acetabular cavity turned towards the outside; a femoral head designed to be fitted inside the said artificial cotyloid cavity and which is one with a femoral shaft designed to be fitted inside the femoral canal, after resection of the head and neck of the femur, and fixed there; the said method characterized by the fact that it involves: the positioning and fixing of the aforementioned cup inside the natural or surgically recreated cotyloid cavity in the pelvis of the subject, with a tubular sheath which is joined to the said cup and turned with its opening towards the outside, within which the said artificial acetabular cavity remains; insertion and fixing of the aforementioned shaft in the femoral canal; housing of the femoral head, joined to the said shaft, inside the said sheath and the aforementioned artificial acetabular cavity; resection of the external part of the said surplus sheath and fixing of its border to an area lying between the said shaft and the said femoral head, thus creating, within the said sheath, an isolated environment designed to cover the joint of the aforementioned prosthesis and to retain within it the fragments of material which are detached from the same joint.

Using the invention an effective lubrication of the joint is achieved without permitting the debris to escape from the sheath.

This is because an environment is created, inside the sheath, which is isolated as compared to the outside and which therefore retains within it any debris which is formed over time around the joint surfaces in mutual contact and subject to friction.

The features of the invention, which do not emerge from the above, are described below, with particular reference to the enclosed drawings, in which:

    - fig. 1 illustrates the device at an intermediate stage of its application following the method in discussion;

- fig.2 illustrates the device in fig. 1 in its final configuration;

- fig.3 illustrates a variation of the device which in this case is fixed to a component of the prosthesis;

- fig.4 illustrates a second type of prosthesis, consisting of a simple cup, and the device in question modified accordingly;

- fig.5 illustrates a variation of the device, fixed to a component of the second type of the prosthesis.

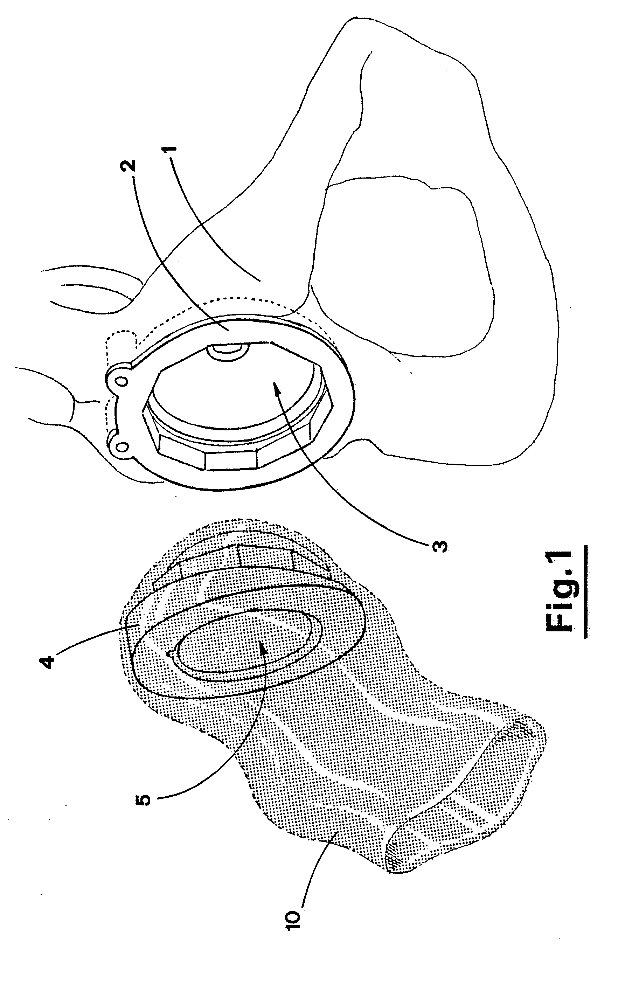

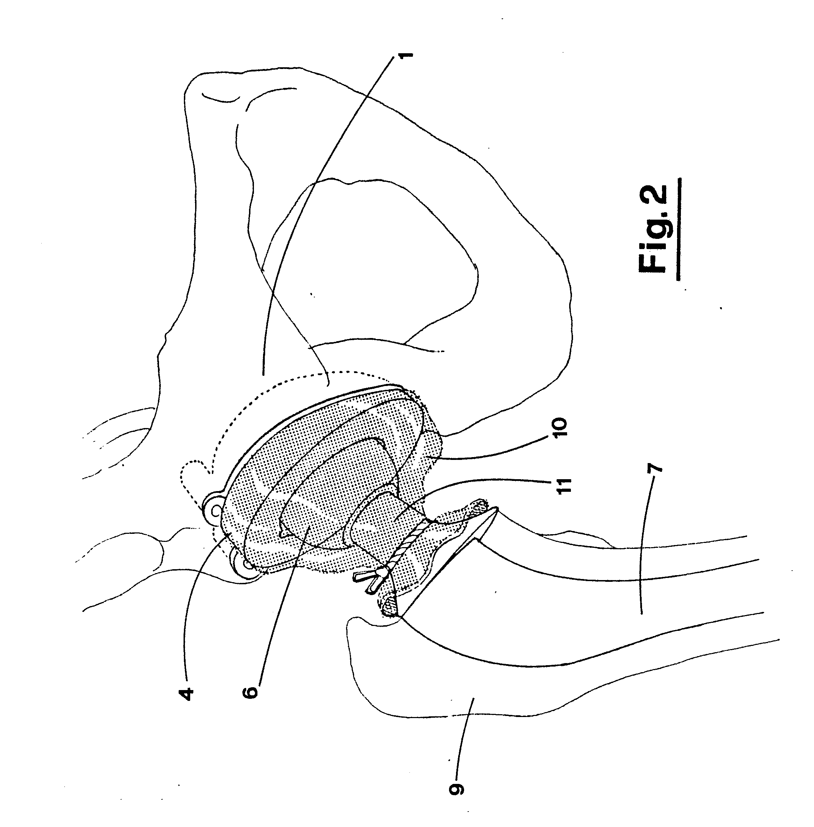

As regards the above figures 1 and 2, 1 is used to indicate a part of the pelvic bone apparatus, in particular the part including the cotyloid or acetabular region, destined to hold a cup 2 which represents the first component of the prosthesis.

The prosthesis which must replace the hip joint consists of a metal cup 2, containing a cavity 3 in which a polyethylene insert 4 may, at least partially, be inserted and fastened by, for example, a join.

The polyethylene insert contains an artificial acetabular cavity 5 which will hold the artificial femoral head 6, for example of the type fixed to the summit of a shaft 7 (fig.2).

The method involves, first of all, the fixing of the cup 2 to the part of the pelvis 1 inside the natural or surgically recreated cotyloid cavity.

In this case it is fixed by means of a press-fit coupling or by screwing the metal cup to the corresponding bone part of the pelvis.

Subsequently, the insert is fitted inside a tubular fabric sheath 10 so that the bottom of the latter remains between the same insert and the cup 2 when these two elements of the prosthesis are fitted together.

The natural head and neck of the femur are resected from the femur 9 and the shaft 7 is fitted inside the femoral canal, after the latter has been suitably prepared, where it is fixed by cementing or a press-fit or with other known techniques.

The head of the artificial femur 6, joined to the shaft 7, is fitted into the sheath 10 and then into the cavity 5.

The surplus external part of the sheath is cut away, so that a suitable length remains, and the border of the sheath is closed by fastening it with a non-absorbable thread to the neck 11 of the shaft 7 (fig.2): to perfect the result of this final stage of the method, a circular groove may be created in the neck 11, or other known means may be used.

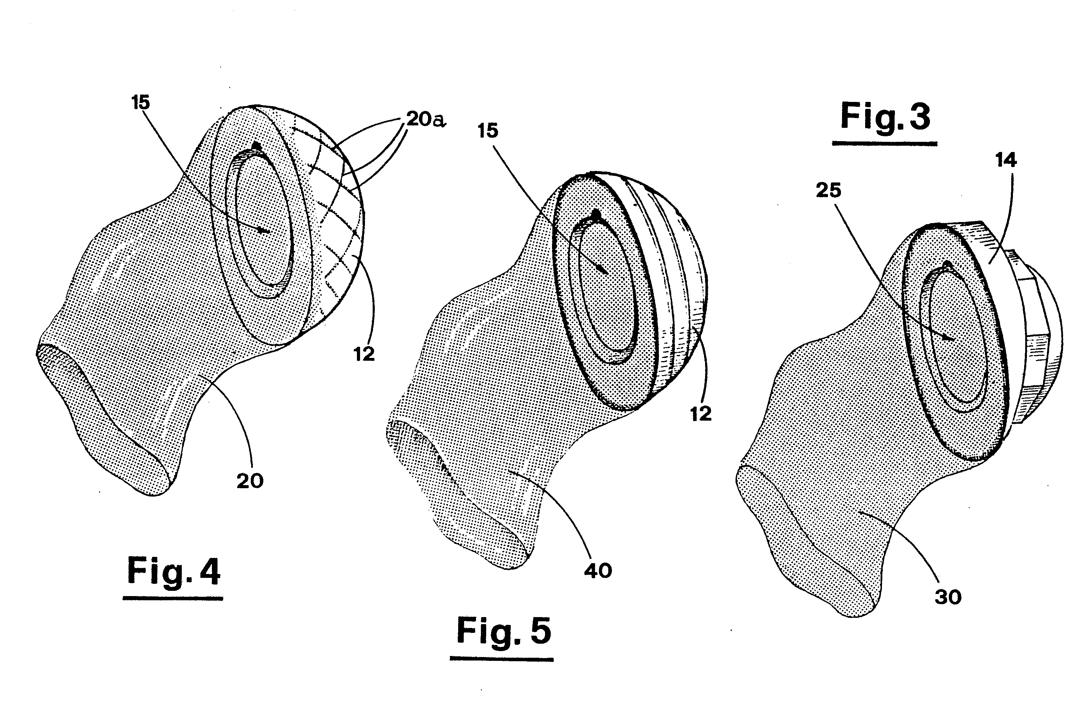

Fig.4 shows a polyethylene cup 12, containing the acetabular cavity 15 and designed to be cemented to the corresponding pelvic bone.

For this second type of prosthesis a different type of sheath is envisaged, indicated with 20 in fig. 4.

The bottom of the sheath 20 consists of a fabric 20a with a wider grain, so that it does not hinder the cementing of the posterior part of the cup.

The variation illustrated in fig. 4 may also be used when a metal cup to be cemented is adopted which has the polyethylene insert already fastened to its inner surface.

Moreover, with a variation in the shape of the sheath, the latter could be created without a bottom, i.e. obtained from a portion of a tube, and fixed with the border of one end, during the production of the same prosthesis, to the circular border of the polyethylene insert 14 which protrudes from the metal cup, as illustrated in fig.3 where the sheath is indicated with 30; or the sheath may be previously fixed to the cup 12, as illustrated in fig.5 where the sheath is indicated with 40.

The sheath is fixed to the neck of the shaft in such a way that the material forming the sheath is not subject to mechanical strain of any kind during the movements of the joint to avoid any breaks or tears.

In particular the sheath may be formed like a corrugated tube so that it is flexible in all the planes.

The material from which the sheath is made is obviously biocompatible: for example materials such as Dacron or Teflon can be used, which have already been widely used in Surgery and secured in such as way as to prevent the escape of any debris.

To allow, however, entry of biological liquid to the environment within the sheath, the sheath may also be made in "Goretex", which is a material capable of allowing the passage of liquid in one direction only, thus from the outside towards the inside, and not in the opposite direction.

In this way an effective lubrication of the joint is achieved without permitting the debris to escape from the sheath.

**Claims**

1) Method for retaining debris detached from total hip prosthesis joints, the latter of the type consisting of: a cup (12) designed to be fixed inside the pelvic cotyloid cavity (1) of the subject fitted with the said prosthesis, containing an artificial acetabular cavity (15) turned towards the outside; a femoral head (6) designed to be fitted inside the said artificial acetabular cavity (15) and which is fixed to a femoral shaft (7) designed to be fitted inside the femoral canal, after resection of the upper part of the femur (9), and fixed there; said method being characterized in that it envisages: positioning and fixing of said cup (12) inside the natural cotyloid cavity of the pelvis (1) of the subject, with a net sheath (20,40) which is attached to said cup (12) and turned with its opening towards the outside, within which said artificial acetabular cavity remains situated (15); fitting and fixing of said shaft (7) in the femoral canal; housing of said femoral head (6), fixed to said shaft (7), inside said sheath (20,40) and said artificial acetabular cavity (15); resection of the surplus part of said sheath (20,40) and fixing of its border to the neck (11) of said shaft (7), with the consequent creation, inside said sheath, of an isolated environment designed to contain the joint of said prosthesis and to retain within it the fragments of material which break away from said joint.

2) Method according to claim 1, characterized in that the bottom (20a) of the said sheath (20), made with a larger grain than the rest of the sheath, it placed between the aforementioned cup (12) and the corresponding bone part to which it is fixed with cement.

3) Method for retaining debris detached from total hip prosthesis joints, the latter of the type consisting of: a cup (2) designed to be fixed inside the pelvic cotyloid cavity (1) of the subject fitted with the said prosthesis, said cup (2) being designed to hold, at least partially, on its inside an insert (4,14), containing an artificial acetabular cavity (5, 25) turned towards the outside; a femoral head (6) designed to be fitted inside said artificial acetabular cavity (5,25) and which is fixed to a femoral shaft (7) designed to be fitted inside the femoral canal, after resection of the upper part of the femur (9), and fixed there; said method being characterized in that it envisages : positioning and fixing of the aforementioned cup (2) inside the natural cotyloid cavity of the pelvis (1) of the subject, turned with its opening towards the outside; placing said insert (4, 14) inside said cup (2), with a sheath (10,30) attached to said insert and turned with its opening towards the outside, within which said artificial acetabular cavity (5,25) remains situated; fitting and fixing of said shaft (7) in the femoral canal; housing of said femoral head (6), fixed to said shaft (7), inside the said sheath (10, 30) and said artificial acetabular cavity (5,25); resection of the surplus part of the said sheath (10,30) and fixing of its border to the neck (11) of said shaft (7), with the consequent creation, inside said sheath, of an isolated environment designed to contain the joint of said prosthesis and to retain within it the fragments of material which break away from said joint.

4) Method according to claim 3, characterized in that the bottom of a sheath (10), made with a homogeneous grain, is placed between the same insert (4) and cup (2).

5) Method according to claim 1, characterized in that said sheath (40), that is made in a generally tubular form, is joined with the border of one end to said cup (12) before fitting together the prosthesis components.

6) Method according to claim 3, characterized in that said sheath (30), that is created in a generally tubular form, is joined with the border of one of its ends to said insert (14) before fitting together the prosthesis components.

7) Method according to claim 1, characterized in that the border of said sheath (40) is joined to the circular border of said cup (2).

8) Device for retaining debris detached from the joints of total hip prostheses of the type consisting of: a cup (12), fixed to the inside of the cotyloid cavity of the pelvis (1) of the subject with the prosthesis, containing an artificial acetabular cavity (15) turned towards the outside; a femoral head (6) fitted inside the artificial acetabular cavity (15) and which is fixed to a femoral shaft (7) fitted in the femoral canal, of the femur (9), from which the upper part has been resected, and fixed there; said device being characterized in that it consists of a tubular sheath (20,40) which is joined to said cup (12) and turned with the opening towards the outside, inside of which said artificial acetabular cavity (15) remains situated; the border of the opening of said sheath (20,40) being closed on said neck (11) of said shaft (7), thus creating, inside the said sheath, an isolated environment designed to contain the joint of said prosthesis and retain within it the fragments of material which are detached from said joint.

9) Device according to claim 8, characterized in that the bottom (20a) of said sheath (20), with a larger grain than the remaining part, is placed between said cup (12) and the corresponding bone part to which it is fixed by cement.

10) Device for the containing and retention of fragments detached from the joints of total hip prostheses of the type consisting of: a cup (2), fixed to the inside of the cotyloid cavity of the pelvis (1) of the subject with the prosthesis, bearing inside it, at least partially, an insert (4,14), containing an artificial acetabular cavity (5,25) turned towards the outside; a femoral head (6) fitted inside the artificial acetabular cavity (5,25) and which is fixed to a femoral shaft (7) fitted in the femoral canal, of the femur (9), from which the upper part has been resected, and fixed there; said device being characterized in that it consists of a tubular sheath (10,30) attached to said insert (4,14) with the opening turned towards the outside, inside of which said artificial acetabular cavity (5,25) remains situated; the border of the opening of said sheath (10,30) being closed on said neck (11) of said shaft (7), thus creating, inside the said sheath, an isolated environment designed to contain the joint of said prosthesis and retain within it the fragments of material which are detached from said joint.

11) Device according to claim 10, characterized in that the bottom of said sheath (10), which is produced with a homogeneous grain, is placed between the same insert (4) and cup (2).

12) Device according to claim 8, characterized in that said sheath (40) is created in a generally tubular shape, with the border of one of its ends joined to said cup (l2).

13) Device according to claim 10, characterized in that said sheath (30) is created in a generally tubular form, with the border of one of its ends joined to said insert (14).

14) Device according to claim 13, characterized in that the border of said sheath (30) is joined to the circular edge of said insert (14).

**Fig.1**

EP 0 346 294 A1

Fig.2

Fig.3

Fig.4

Fig.5

European Patent Office

**PARTIAL EUROPEAN SEARCH REPORT**

. ~ich under Rule 45 of the European Patent Convention shall be considered, for the purposes of subsequent proceedings, as the European search report

Application number

EP 89 83 0229

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | US-A-4 731 088 (COLLIER) <br><br> * Whole document * | 8,10, 12 | A 61 F 2/32 |
| Y | | 9,11, 13,14 | |
| | -- | | |
| Y | US-A-3 864 758 (YAKICH) <br><br> * Abstract; claim 1; figures * | 9,11, 13,14 | |
| | ------ | | |

**TECHNICAL FIELDS SEARCHED (Int. Cl.4)**

A 61 F

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 8-14
Claims searched incompletely:
Claims not searched: 1-7
Reason for the limitation of the search:

Method for treatment of the human or animal body by surgery or therapy (see art. 52(4) of the European Patent Convention).

| Place of search | Date of completion of the search | Examiner |
|---|---|---|
| The Hague | 18-08-1989 | SANCHEZ SANCHEZ |